# EUROPÄISCHE PATENTSCHRIFT

(11) **EP 0 438 465 B1**
(45) Veröffentlichungstag und Bekanntmachung des Hinweises auf die Patenterteilung: **11.01.1995**
(21) Anmeldenummer: 89911543.0
(22) Anmeldetag: 06.10.1989
(51) Int. Cl.: G01N 21/21, G01N 33/66

(54) **VERFAHREN UND VORRICHTUNG ZUR QUANTITATIVEN BESTIMMUNG OPTISCH AKTIVER SUBSTANZEN**
PROCESS AND DEVICE FOR QUANTITATIVE DETECTION OF OPTICALLY ACTIVE SUBSTANCES
PROCEDE ET DISPOSITIF DE DETECTION QUANTITATIVE DE SUBSTANCES OPTIQUEMENT ACTIVES

(30) Priorität: 07.10.1988 DE 3834160; 13.03.1989 DE 3908114
(43) Veröffentlichungstag der Anmeldung: 31.07.1991
(73) Patentinhaber: Siemens-Elema AB, 171 95 Solna 1 (SE); SIEMENS AKTIENGESELLSCHAFT, 80333 München (DE)
(72) Erfinder: SCHMIDTKE, Gerhard, D-7800 Freiburg (DE); RIEDEL, Wolfgang, D-7844 Neuenburg (DE); WOLF, Helmut, W-7802 Metzenhausen (DE)
(86) Internationale Anmeldenummer: EP8901175
(87) Internationale Veröffentlichungsnummer: WO9004163

(56) Entgegenhaltungen:
- EP-A- 0 030 610
- DE-A- 2 724 543
- DE-C- 2 944 113
- US-A- 4 040 718
- US-A- 4 105 337

## Beschreibung

Die Erfindung betrifft ein Verfahren und eine Vorrichtung zur quantitativen Bestimmung der Konzentration optisch aktiver Substanzen, insbesondere von Glukose in der Körperflüssigkeit eines Patienten, durch Polarimetrie unter Erzeugung eines Vergleichssignals, insbesondere eines Differenz- oder Quotientensignals, mit einer ersten Lichtquelle, mit der ein erstes linear polarisiertes Lichtbündel erzeugt wird, das die zu analysierende Substanz durchstrahlt und über einen Analysator einen Detektor beaufschlagt.

Aus der DE-OS 29 44 113 und aus der EP-A 0 030 610 sind Verfahren und Vorrichtungen gemäß dem Oberbegriff des Patentanspruchs 1 bekannt. Bei diesen Verfahren wird die Probe der optisch aktiven Substanz mit linear polarisiertem Licht bestrahlt, das durch einen Faradaymodulator hindurch getreten ist. Nach diesem Durchgang wird der Lichtstrahl in einen Meß- bzw. Referenzstrahl aufgeteilt, die beide einzeln von einem Detektor nachgewiesen werden, wobei aus den erhaltenen Signalen ein Quotienten- oder Differenzsignal erzeugt wird, welches zur quantitativen Bestimmung einer optisch aktiven Substanz in der durchstrahlten Probe herangezogen wird.

Die verfahrensgemäße Einrichtung verfügt über einen komplizierten optischen Aufbau, dessen Miniaturisierung einen erheblichen Aufwand erfordert. Bei dem Verfahren nach dem Stand der Technik wird ein Faraday-Modulator eingesetzt, dessen ein Magnetfeld erzeugende Einrichtung eine große Leistungsaufnahme aufweist, und so nur kurze unabhängige Betriebszeiten innerhalb eines lebenden Körpers ermöglichen.

Ausgehend von diesem Stand der Technik liegt der Erfindung die Aufgabe zugrunde, ein Verfahren und eine Vorrichtung zur quantitativen Bestimmung optisch aktiver Substanzen zu schaffen, die eine einfache Miniaturisierung mit einer geringen Leistungsaufnahme und dadurch eine langzeitig wirksame Implantierung in einem lebenden Körper gestatten.

Diese Aufgabe wird gemäß der Erfindung für ein Verfahren durch die kennzeichnenden Merkmale des Anspruchs 1 gelöst. Eine Vorrichtung zur Durchführung des Verfahrens ist in Anspruch 5 gekennzeichnet.

Die Verwendung von zwei verschieden linear polarisierten Lichtstrahlen, die von zwei Halbleiterlichtquellen in einfacher und stromsparender Weise erzeugt werden können, deren Lichtemission in zeitlichem Wechsel mit einer Umschaltfrequenz gesteuert wird und deren feste Polarisationsrichtungen um einen vorbestimmten Winkel voneinander abweichen, gestatten ein hohes Auflösungsvermögen bei der Konzentrationsbestimmung von optisch aktiven Substanzen in einer durchstrahlten Lösung bei kleiner Leistungsaufnahme der elektrischen und elektronischen Komponenten.

Der starre Aufbau ohne mechanisch bewegte Teile mit zwei festen Strahlengängen gestattet ein hohes Signal/Rauschverhältnis.

Die Verwendung eines phasenempfindlichen Nachweises der Meßsignale durch eine Lock-In-Technik erhöht weiter das Signal/Rauschverhältnis.

Die Verwendung von Lumineszenzdioden oder Halbleiterlaserdioden erlaubt die platzsparende Integrierbarkeit auf Schaltplatinen.

Nachfolgend werden vier Ausführungsbeispiele der Erfindung anhand der Zeichnung näher erläutert. Es zeigen:
- Fig. 1: eine schematische Ansicht einer Vorrichtung zur quantitativen Bestimmung optisch aktiver Substanzen gemäß einem ersten Ausführungsbeispiel,
- Fig. 2: einen Querschnitt entlang der Linie II-II gemäß Fig. 1 des ersten Ausführungsbeispiels,
- Fig. 3: den Verlauf des von einem Detektor erzeugten Photostrom in Abhängigkeit der Polarisationsrichtungen von einfallendem Licht,
- Fig. 4: eine schematische Ansicht einer Vorrichtung zur quantitativen Bestimmung optisch aktiver Substanzen gemäß einem zweiten Ausführungsbeispiel und
- Fig. 5a & 5b: eine schematische Draufsicht und eine schematische Seitenansicht einer Vorrichtung zur quantitativen Bestimmung optisch aktiver Substanzen gemäß einem dritten Ausführungsbeispiel und
- Fig. 6: eine schematische Seitenansicht einer Vorrichtung zur quantitativen Bestimmung optisch aktiver Substanzen gemäß einem vierten Ausführungsbeispiel.

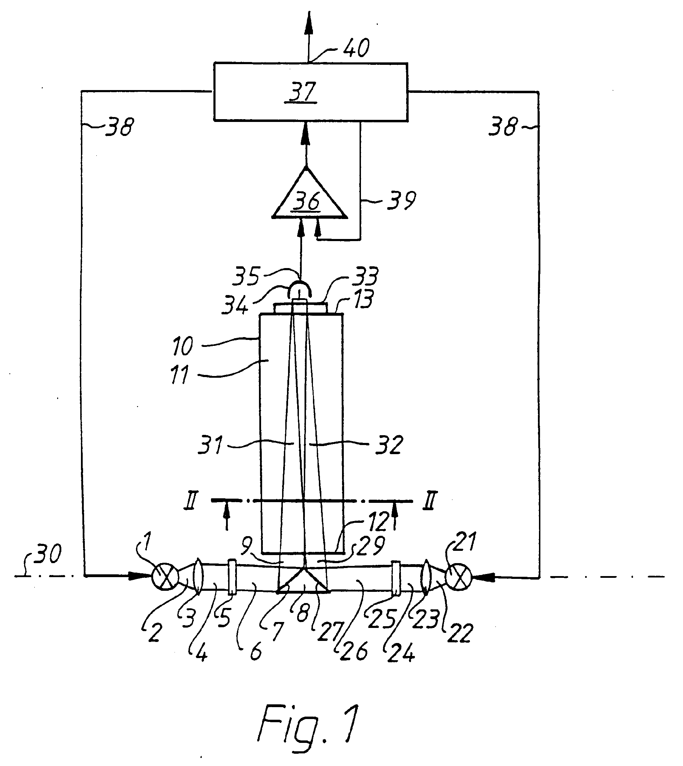

Die Figur 1 zeigt schematisch eine Vorrichtung zur quantitativen Bestimmung optisch aktiver Substanzen in einer menschlichen Körperflüssigkeit gemäß einem ersten Ausführungsbeispiel der Erfindung. Die Vorrichtung weist eine erste Lumineszenzdiode 1 und eine zweite Lumineszenzdiode 21 auf, die Licht der im wesentlichen selben Wellenlänge emittieren. Der Wellenlängenbereich des emittierten Lichtes kann zwischen 400 Nanometer und 900 Nanometer gewählt werden. Die beiden Lumineszenzdioden 1 und 21 können auch durch Halbleiterlaser ersetzt werden, die im gleichen Wellenlängenbereich arbeiten.

In der Figur 1 wird zur Veranschaulichung davon ausgegangen, daß beide Lumineszenzdioden 1 und 21 gleichzeitig betrieben werden. Dieses geschieht in der Anwendung der beschriebenen Vorrichtung nur zu Justier- oder Prüfzwecken, im Meßbetrieb strahlen die beiden Lumineszenzdioden 1 und 21 intermittierend.

Die erste Lumineszenzdiode 1 erzeugt einen ersten divergenten Lichtstrahl 2. Der erste divergente Lichtstrahl 2 wird durch eine erste Kollimatorlinse 3 in einen ersten parallelen Lichtstrahl 4 kollimiert.

Der erste parallele Lichtstrahl 4 durchquert ein erstes Polarisationsfilter 5, welches das Licht, das von der ersten Lumineszenzdiode 1 ausgestrahlt wird, linear polarisiert. Beim Einsatz von Laserdioden wird das Laserlicht der die erste Lumineszenzdiode 1 ersetzende Laserdiode durch das erste Polarisationsfilter 5 nachpolarisiert. Die Polarisation des durch das erste Polarisationsfilter 5 hindurchtretenden ersten polarisierten Lichtstrahls 6 soll ein Polarisationsverhältnis von möglichst 100:1 aufweisen.

Der erste polarisierte Lichtstrahl 6 beaufschlagt einen ersten kollimierenden Spiegel 7, der auf einem Stützkeil 8 angeordnet ist. Der erste kollimierende Spiegel 7 lenkt den ersten polarisierten Lichtstrahl 6 in einen ersten konvergenten Lichtstrahl 9 um.

Der erste konvergente Lichtstrahl 9 tritt durch eine Küvette 10 hindurch, die mit dem zu analysierenden Dialysat 11 gefüllt ist. Die Küvette 10 ist quaderförmig mit einer rechteckigen Vorderwand 12 und einer rechteckigen Rückwand 13. Die Vorderwand 12 der Küvette 10 ist für die Wellenlänge des emittierten Lichtes der beiden Lumineszenzdioden 1 und 21 transparent. Auch die Rückwand 13 der Küvette 10 ist transparent ausgeführt. Die Küvette 10 verfügt weiterhin vorzugsweise über zwei in der Zeichnung nicht dargestellte Bohrungen, jeweils eine in der Nähe der Vorderwand 12 und der Rückwand 13, zur Durchströmung mit dem Dialysat 11.

Die zweite Lumineszenzdiode 21 erzeugt einen zweiten divergenten Lichtstrahl 22. Der zweite divergente Lichtstrahl 22 wird durch eine zweite Kollimatorlinse 23 in einen zweiten parallelen Lichtstrahl 24 kollimiert.

Der zweite parallele Lichtstrahl 24 durchquert ein zweites Polarisationsfilter 25, welches das Licht, das von der zweiten Lumineszenzdiode 21 ausgestrahlt wird, linear polarisiert. Beim Einsatz von Laserdioden wird das Laserlicht der die zweite Lumineszenzdiode 21 ersetzenden Laserdiode durch das zweite Polarisationsfilter 25 nachpolarisiert. Die Polarisation des durch das zweite Polarisationsfilter 25 hindurchtretenden zweiten polarisierten Lichtstrahls 26 soll ein dem ersten polarisierten Lichtstrahl 6 entsprechendes Polarisationsverhältnis aufweisen.

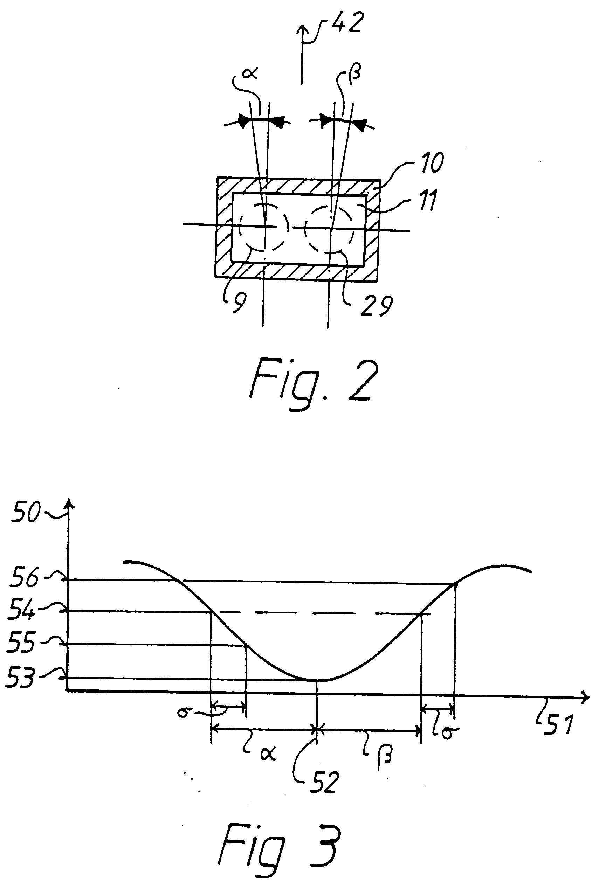

Die genaue Polarisationsrichtung des zweiten polarisierten Lichtstrahls 26 im Verhältnis zu der Polarisationsrichtung des ersten polarisierten Lichtstrahls 6 wird später im Zusammenhang mit der Figur 2 erläutert.

Der zweite polarisierte Lichtstrahl 26 beaufschlagt einen zweiten kollimierenden Spiegel 27, der gegenüber dem ersten kollimierenden Spiegel 7 auf dem Stützkeil 8 angeordnet ist. Der zweite kollimierende Spiegel 27 lenkt den zweiten polarisierten Lichtstrahl 26 in einen zweiten konvergenten Lichtstrahl 29 um.

Der zweite konvergente Lichtstrahl 29 tritt ebenfalls durch die Küvette 10 hindurch, die mit dem zu analysierenden Dialysat 11 gefüllt ist.

Die beiden Lumineszenzdioden 1 und 21 und die von ihnen ausgehenden Lichtstrahlen 2, 4, 6, 22, 24 und 26 sind fluchtend bezüglich einer Achse 30 ausgerichtet. Die Strahltaille der beiden polarisierten Lichtstrahlen 6 und 26 ist im wesentlichen gleich groß. Die beiden kollimierenden Spiegel 7 und 27 weisen die gleichen Abbildungseigenschaften auf.

Die Symmetrieebene des ersten kollimierenden Spiegels 7 bildet mit der Symmetrieebene des zweiten kollimierenden Spiegels 27 einen stumpfen Winkel, der nur etwas größer als ein rechter Winkel ist, so daß der erste konvergente Lichtstrahl 9 und der zweite konvergente Lichtstrahl 29 durch die Vorderwand 12 der Küvette 10 derart hindurchtreten, daß sie im wesentlichen durch denselben Flächenanteil der Rückwand 13 aus der Küvette 10 austreten, nachdem der erste konvergente Lichtstrahl 9 eine erste Volumenmenge 31 an Dialysat 11 und der zweite konvergente Lichtstrahl 29 eine zweite Volumenmenge 32 an Dialysat 11 durchströmt hat und wobei vorzugsweise, was nicht in der Zeichnung dargestellt ist, die erste Volumenmenge 31 mit der zweiten Volumenmenge 32 einen möglichst großen gemeinsamen Anteil aufweist.

Die beiden konvergenten Lichtstrahlen 9 und 29 fallen durch die transparente Rückwand 13 der Küvette 10 auf einen Analysator 33 und werden schließlich auf einem Detektor 34 abgebildet.

In einer vereinfachten Ausführung sind die Spiegel 7 und 27 plan ausgeführt und die Brennweiten der Kollimatorlinsen 3 und 23 derart gewählt, daß das divergente Licht der Lumineszenzdioden 1 und 21 konvergent auf den Detektor 34 analog zur Strahlführung 31, 32 abgebildet wird.

Die Polarisationsrichtung der vom Analysator 33 hindurchgelassenen Strahlung wird weiter unten im Zusammenhang mit der Figur 2 angegeben.

Mit der Abbildung der beiden Lumineszenzdioden 1 und 21 auf den Detektor 34 erzeugt man durch die wirkungsvolle Ausnutzung der eingesetzten Lichtquellen ein maximales Photosignal 35 in dem Detektor 34. Das Photosignal 35 beaufschlagt einen Lock-In-Verstärker 36, der ausgangsmäßig mit einem Eingang einer Steuer- und Auswerteschaltung 37 verbunden ist. Die Steuer- und Auswerteschaltung 37 steuert die beiden Lumineszenzdioden 1 und 21 über Steuerleitungen 38 an und gibt über eine Referenzsteuerleitung 39 ein Referenzsignal für den Lock-In-Verstärker 36. Ein in der Steuer- und Auswerteschaltung 37 erzeugtes Regelsignal 40 steht für die Steuerung einer in der Zeichnung nicht dargestellten Insulinpumpe oder für eine externe Anzeige zur Verfügung.

Zur Veranschaulichung der Ansteuerung der beiden Lumineszenzdioden 1 und 21 sowie der Signalverarbeitung des Photosignales 35 des Detektors 34 in dem Lock-In-Verstärker 36 und der Steuer- und Auswerteschaltung 37 wird zuerst die Ausrichtung der verschiedenen Polarisationsrichtungen der beiden Polarisationsfilter 5 und 25 sowie des Analysators 33 untereinander mit Hilfe der Figur 2 erläutert.

Die Figur 2 zeigt einen Querschnitt entlang der Linie II-II in Figur 1. Die küvette 10 ist zuerst mit einem optisch nicht aktiven Dialysat 11 gefüllt und wird von den beiden konvergenten Lichtstrahlen 9 und 29 durchstrahlt, die zu ihrer Verdeutlichung durch zwei strich-linierte Kreise angedeutet sind, die erheblich voneinander entfernt sind. Vorzugsweise überlappen sich allerdings die Bereiche der konvergenten Lichtstrahlen 9 und 29 auch schon nahe der Vorderwand 12 der Küvette 10.

Durch den Pfeil 42 ist eine Polarisationsrichtung für Licht in der Ebene des Querschnittes durch die Küvette 10 vorgegeben. Das erste Polarisationsfilter 5 ist derart angeordnet, daß die Polarisation des ersten konvergenten Lichtstrahls 9 gegenüber der durch den Pfeil 42 vorgegebenen Polarisationsrichtung um einen kleinen vorbestimmten Winkel α verdreht ist. Das zweite Polarisationsfilter 25 ist derart angeordnet, daß die Polarisation des zweiten konvergenten Lichtstrahls 29 gegenüber der durch den Pfeil 42 vorgegebenen Polarisationsrichtung um einen kleinen vorbestimmten Winkel β verdreht ist. In der Figur 2 weisen die Winkel α und β von dem Pfeil 42 weg. In einer anderen Ausgestaltung der Vorrichtung können die Winkel α und β auch in eine von dem Pfeil 42 aus gesehen gleiche Richtung weisen; die durch die Winkel α und β vorgegebenen, von der durch den Pfeil 42 vorgegebenen Polarisationsrichtung abweichenden Polarisationsrichtungen der ersten und zweiten konvergenten Lichtstrahlen 9 und 29 müssen nur verschieden sein.

Die Polarisationsrichtung des im Querschnitt der Figur 2 nicht dargestellten Analysators 33 ist im wesentlichen rechtwinklig zu der durch den Pfeil 42 vorgegebenen Richtung.

Zur Erläuterung der verschiedenen von dem Detektor 34 aufgenommenen Meßsignale wird im Folgenden Bezug auf die Figur 3 genommen. Die Figur 3 zeigt den Verlauf des von dem Detektor 34 erzeugten Photostroms 50 in Abhängigkeit der Polarisationsrichtung und des damit gegebenen Polarisationswinkels 51 von einfallendem Licht für eine sehr kleine Depolarisation.

Der Nullwinkel 52 wird durch die Polarisationsrichtung definiert, für die ein auf den Detektor 34 einfallender linear polarisierter Lichtstrahl einen minimalen Photostrom 53 in dem Detektor 34 erzeugt. Die Größe des minimalen Photostroms 53 ist ein Maß für die Depolarisation des einfallenden unvollständig linear polarisierten Lichtstrahls. Bei einem im wesentlichen rechtwinklig zu dem Pfeil 42 angeordneten Analysator 33 wird der minimale Photostrom 53 durch einfallendes Licht erzeugt, das in Richtung des Pfeils 42 polarisiert ist.

Der erste konvergente Lichtstrahl 9 aus der Figur 2 weist eine Polarisation auf, deren Richtungswinkel um den Winkel α kleiner als der Richtungswinkel des Pfeils 42 ist, wenn Winkel bezüglich dem Pfeil 42 im Uhrzeigersinn gemessen werden. Der zweite konvergente Lichtstrahl 29 weist eine Polarisation auf, deren Richtungswinkel demgemäß um den Winkel β größer ist. Die Winkel α und β liegen im Bereich von wenigen Grad.

Bei einem optisch nicht drehenden Dialysat 11 in der Küvette 10 und bei betragsmäßig gleichem Winkel |α|=|β| ist dann der im Detektor 34 erzeugte Justierphotostrom 54 gleich. Das Differenzsignal der Justierphotoströme 54 ist null und ein Quotientensignal ist gleich eins.

Zum Nachweis der Justierphotoströme 54 versorgt die Steuer- und Auswerteschaltung 37 die Lumineszenzdioden 1 und 21 über die Steuerleitungen 38 abwechselnd mit Strom, so daß die Lumineszenzdioden 1 und 21 mit einer Umschaltfrequenz aus einem Bereich von z.B. zehn Hertz bis tausend Hertz abwechselnd Licht emittieren. Die Emission des Lichts ist vorzugsweise noch mit einer zweiten Modulation überlagert, die mit einer Frequenz aus einem Bereich von z.B. ein Kilohertz bis hundert Kilohertz durchgeführt wird.

Durch die abwechselnde Emission der beiden Lumineszenzdioden 1 und 21 werden nacheinander die beiden Richtungswinkel α und β ausgemessen, die bei mit nicht drehendem Dialysat 11 gefüllter Küvette 10 den gleichen Justierphotostrom 54 hervorrufen. In der Steuer- und Auswerteschaltung 37 wird das in der einen Halbperiode der Umschaltfrequenz gemessene Signal zwischengespeichert und mit dem in der zweiten Halbperiode gemessenen Signal z. B. in einem Komparator verglichen. Zur Erhöhung des Signal/Rauschverhältnisses ist die Lichtemission der beiden Lumineszenzdioden 1 und 21 jeweils mit der zweiten Modulation überlagert, die einen phasenempfindlichen Nachweis der gemessenen Signale mit dem Lock-In-Verstärker 36 ermöglicht.

Ein optisch drehendes Dialysat 11 dreht die lineare Polarisation der durch die Küvette 10 hindurch laufenden konvergenten Lichtstrahlen 9 und 29 jeweils gleichsinnig um einen Winkel σ weiter. Die Lichtemission der ersten Lumineszenzdiode 1 erzeugt einen ersten Signalphotostrom 55, der dem Polarisationswinkel α+σ zugeordnet ist. Die Lichtemission der zweiten Lumineszenzdiode 21 erzeugt einen zweiten Signalphotostrom 56, der dem Polarisationswinkel β+σ zugeordnet ist. Der Summenwinkel α+σ liegt näher am Nullwinkel 52 als der Summenwinkel α+β. Der erste Signalphotostrom 55 des Summenwinkels α+σ ist kleiner als der Justierphotostrom 54 , der zweite Signalphotostrom 56 des Summenwinkels α+β ist größer als der Justierphotostrom 54.

Die in der ersten respektive zweiten Halbperiode der Umschaltfrequenz der Lichtemission der Lumineszenzdioden 1 und 21 erzeugten ersten respektive zweiten Signalphotoströme 55 bzw. 56 sind ungleich. Der Gehalt an optisch drehenden Substanzen im Dialysat 11 kann mit ihnen ermittelt werden; z.B ist das Differenzsignal aus dem zwischengespeicherten ersten Signalphotostrom 55 und dem zweiten Signalphotostrom 56 ungleich null und ein Quotientensignal weicht von dem Wert eins ab. Auch jedes andere Vergleichssignal, welches aus dem zwischengespeicherten ersten Signalphotostrom 55 und dem zweiten Signalphotostrom 56 gebildet wird, kann zur Bestimmung der Konzentration an optisch aktiven Substanzen in dem Dialysat 11 benutzt werden.

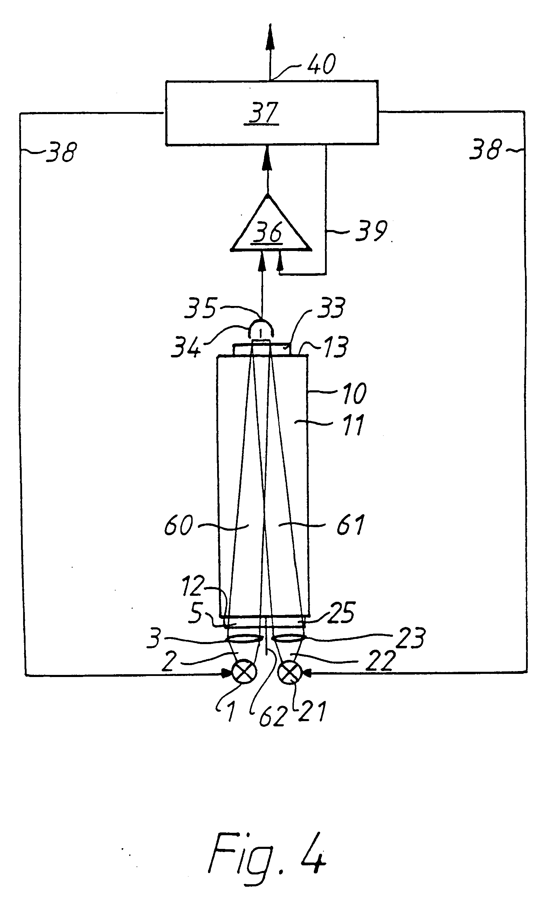

Die Figur 4 stellt schematisch eine Ansicht einer Vorrichtung zur quantitativen Bestimmung optisch aktiver Substanzen gemäß einem zweiten Ausführungsbeispiel dar. Die Vorrichtung weist eine erste Lumineszenzdiode 1 und eine zweite Lumineszenzdiode 21 auf, die wie im ersten Ausführungsbeispiel ausgewählt werden.

Auch in der Figur 4 wird zur Veranschaulichung davon ausgegangen, daß beide Lumineszenzdioden 1 und 21 gleichzeitig betrieben werden. Dieses geschieht in der Anwendung der beschriebenen Vorrichtung nur zu Justier- oder Prüfzwecken, im Meßbetrieb strahlen die beiden Lumineszenzdioden 1 und 21 intermittierend.

Die erste Lumineszenzdiode 1 erzeugt einen ersten divergenten Lichtstrahl 2. Der erste divergente Lichtstrahl 2 wird durch eine erste Kollimatorlinse 3 kollimiert und durchquert ein erstes Polarisationsfilter 5, welches das Licht, das von der ersten Lumineszenzdiode 1 ausgestrahlt wird, linear polarisiert. Beim Einsatz von Laserdioden wird das Laserlicht der die erste Lumineszenzdiode 1 ersetzende Laserdiode durch das erste Polarisationsfilter 5 nachpolarisiert. Die Polarisation des durch das erste Polarisationsfilter 5 hindurchtretenden ersten abbildenden Lichtstrahls 60 soll ein Polarisationsverhältnis von möglichst 100:1 aufweisen.

Der erste abbildende Lichtstrahl 60 tritt durch die Küvette 10 hindurch, die mit dem zu analysierenden Dialysat 11 gefüllt ist. Die Ausgestaltung der Küvette 10 ist dem ersten Ausführungsbeispiel entnommen.

Die zweite Lumineszenzdiode 21 erzeugt einen zweiten divergenten Lichtstrahl 22. Der zweite divergente Lichtstrahl 22 wird durch eine zweite Kollimatorlinse 23 kollimiert, und durchquert ein zweites Polarisationsfilter 25, welches das Licht, das von der zweiten Lumineszenzdiode 21 ausgestrahlt wird, linear polarisiert. Beim Einsatz von Laserdioden wird das Laserlicht der die zweite Lumineszenzdiode 21 ersetzende Laserdiode durch das zweite Polarisationsfilter 25 nachpolarisiert. Die Polarisation des durch das zweite Polarisationsfilter 25 hindurchtretenden zweiten abbildenden Lichtstrahls 61 soll ein dem ersten abbildenden Lichtstrahl 60 entsprechendes Polarisationsverhältnis aufweisen.

Die genaue Polarisationsrichtung des zweiten abbildenden Lichtstrahls 61 im Verhältnis zu der Polarisationsrichtung des ersten abbildenden Lichtstrahls 60 ist oben im Zusammenhang mit der Figur 2 erläutert. Dabei entsprechen die dort mit den Bezugszeichen 9 und 29 versehenen Lichtstrahlen nun den Lichtstrahlen 60 und 61.

Der zweite abbildende Lichtstrahl 61 tritt ebenfalls durch die Küvette 10 hindurch, die mit dem zu analysierenden Dialysat 11 gefüllt ist.

Die beiden Lumineszenzdioden 1 und 21 und die von ihnen ausgehenden Lichtstrahlen sind im wesentlichen parallel zur Normalen auf die Vorderwand 12 der Küvette 10 ausgerichtet.

Das erste Polarisationsfilter 5 und das zweite Polarisationsfilter 25 sind so angeordnet, daß kein Licht der ersten Lumineszenzdiode 1 auf das zweite Polarisationsfilter 25 und kein Licht der zweiten Lumineszenzdiode 21 auf das erste Polarisationsfilter 5 fallen kann. Dies kann durch eine optionale Trennwand 62 erreicht werden. Die beiden Kollimatorlinsen 3 und 23 können durch eine einzige Kollimatorlinse ersetzt werden, die die Abbildungseigenschaften der Einzellinsen vereint.

Die beiden abbildenden Lichtstrahlen 60 und 61 fallen durch die transparente Rückwand 13 der Küvette 10 auf den Analysator 33 und werden schließlich auf dem Detektor 34 abgebildet.

Das Photosignal 35 des Detektors 34 wird in dem Lock-In-Verstärker 36 und in der Steuer- und Auswerteschaltung 37 analog dem ersten Ausführungsbeispiel verarbeitet. Auch erfolgt die Ansteuerung der beiden Lumineszenzdioden 1 und 21 über die Steuerleitungen 38 in derselben Weise wie beim ersten Ausführungsbeispiel.

In einer bevorzugten Ausführungsform verfügt die Küvette 10 über eine in der Figur 2 vergrößert dargestellte Querschnittsfläche, deren Seitenlängen drei Millimeter bzw. sechs Millimeter betragen. Der Durchmesser der in ihr verlaufenden beiden konvergenten Lichtstrahlen 9 und 29, bzw der beiden abbildenden Lichtstrahlen 60 und 61, beträgt ungefähr zwei Millimeter. Die Länge der Küvette 10 beträgt einige Zentimeter. Der transparente Teil der Vorderwand 12 der Küvette kann aus einem Neutralglasfenster oder aus den beiden Polarisationsfiltern 5 und 25 gebildet sein. Der transparente Teil der Rückwand 13 der Küvette wird vorzugsweise aus dem Analysator 33 aufgebaut. Die Kollimatorlinsen 3 und 23 sind mit den Lumineszenzdioden 1 und 21 einerseits und den Polarisationsfiltern 5 und 25 andererseits mit Immersionsschichten verbunden und gekittet, so daß die Reflexionsverluste im Strahlengang niedrig gehalten werden und der gesamte optische Aufbau klein und mechanisch haltbar ist.

Mit dem ohne große Zwischenräume ausgeführten, mit der Küvette 10 fluchtenden optischen Aufbau gemäß dem zweiten Ausführungsbeispiel, sowie mit der in Verlängerung der Küvette 10 angeordneten Meß- und Steuerelektronik in integrierter Form, die aus den Komponenten 34, 36 und 37 aufgebaut ist, ergibt sich mit z.B. einer Minibatterie zur Energieversorgung eine zur Implantierung in einen Patienten hervorragend geeignete Meßeinrichtung zur Bestimmung der Konzentration von optisch drehenden Substanzen, insbesondere der zeitlichen Veränderung der Konzentration solcher Substanzen.

Die Steuer- und Auswerteschaltung 37 kann eine Insulinpumpe steuern. Das Regelsignal 40 der Steuer- und Auswerteschaltung 37 kann ein Null-Eins-Signal sein, das die Insulinpumpe in Bewegung setzt oder stoppt. Es kann weiterhin ein kontinuierliches Signal sein, das entweder der Anzeige der Konzentration einer optisch drehenden Substanz dient oder das eine kontinuierlich arbeitende Pumpe steuert.

Durch entsprechende Zeitglieder kann die Konzentrationsveränderung von anderen optisch aktiven Stoffen, deren Veränderung z.B. langsamer als die des gesuchten Stoffes erfolgt, herausgefiltert werden. Zur Erhöhung des Dynamikbereichs können noch mehr Lumineszenzdioden vorgesehen sein, die jeweils paarweise einander zugeordnet sind und deren Lichtemission im Querschnitt der Küvette 10 durch ein Polarisationsfilter jeweils eine etwas andere Polarisationsrichtung aufweist. Dies gelingt in besonders einfacher Weise im zweiten Ausführungsbeispiel. Mit einer Rechteckmodulation der Lichtemission der Lumineszenzdioden in drei oder mehr möglichst gleich langen Takten können drei oder mehr Photoströme 50 für die verschiedenen Polarisationswinkel 51 in Figur 3 erfaßt werden. Durch paarweise Auswahl von geeigneten Signalen wird der Dynamikbereich der Konzentrationsmessung erheblich erhöht.

In dem erläuterten ersten bzw. zweiten Ausführungsbeispiel können die Winkel α und β auch |α| = |β| = 45° betragen. Dann kann der Analysator 33 sowohl in der beschriebenen Weise rechtwinklig zur Richtung des in Figur 2 gezeichneten Pfeils 42 als auch in Pfeilrichtung angeordnet werden, was in Abwesenheit von optisch aktiven Stoffen im Meßweg den Abgleich der Lichtquellen gestattet, sofern sich die optischen Lichtwege bezüglich ihrer Transmission bzw. die emittierten Intensitäten der Lichtquellen nicht voneinander unterscheiden.

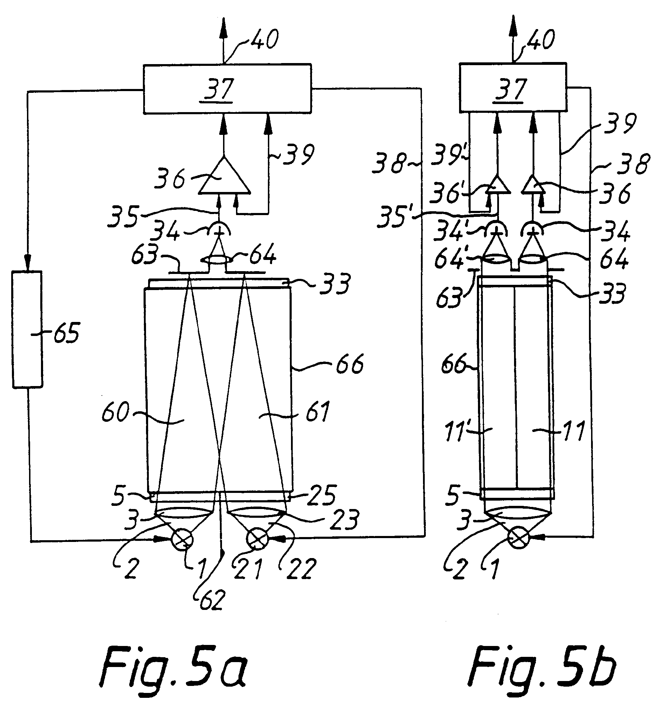

Die Figuren 5a und 5b zeigen schematisch eine Vorrichtung zur quantitativen Bestimmung optisch aktiver Substanzen in einer menschlichen Körperflüssigkeit gemäß einem dritten Ausführungsbeispiel der Erfindung. Im Vergleich zum zweiten Ausführungsbeispiel ist das dritte Ausführungsbeispiel durch einen in der Figur 5b gezeichneten Referenzstrahl und einen Referenzdetektor 34′ erweitert worden.

Die Figur 5b zeigt eine Seitenansicht auf eine Doppelkammerküvette 66, deren zwei Kammern die Meßflüssigkeit oder Dialysat 11 bzw. eine Referenzflüssigkeit 11′ enthalten. Die in der Figur 5a gezeichnete erste Lumineszenzdiode 1 bzw. zweite Lumineszenzdiode 21 erzeugen den ersten divergenten Lichtstrahl 2 bzw. den zweiten divergenten Lichtstrahl 22, die durch die erste Kollimatorlinse 3 bzw. durch die zweite Kollimatorlinse 23 zu dem ersten abbildenden Lichtstrahl 60 und dem zweiten abbildenden Lichtstrahl 61 fokussiert werden. Die Lichtstrahlen 60 und 61 durchstrahlen die zwei Kammern der Doppelkammerküvette 66, was in Seitenansicht der Figur 5b deutlich zu erkennen ist.
Ein Teil des Lichts, das jeweils das Dialysat 11 bzw. die Referenzflüssigkeit 11′ parallel durchstrahlt, wird mit Hilfe einer Aperturblende 63 ausgeblendet. Der verbliebene Anteil der Lichtstrahlen 60 und 61, die durch das Dialysat 11 hindurchgetreten sind, wird mit Hilfe einer Sammellinse 64 auf den Detektor 34 abgebildet. Der verbliebene Anteil der Lichtstrahlen 60 und 61, die durch die Referenzflüssigkeit 11′ hindurchgetreten sind, wird mit Hilfe einer zweiten Sammellinse 64′ auf den Referenzdetektor 34′ gelenkt.

Der Detektor 34 erzeugt das Photosignal 35, das den Lock-In-Verstärker 36 beaufschlagt, der ausgangsmäßig mit einem Eingang der Steuer- und Auswerteschaltung 37 verbunden ist. Die Steuer- und Auswerteschaltung 37 steuert die beiden Lumineszenzdioden 1 und 21 über Steuerleitungen 38 an und gibt über die Referenzsteuerleitung 39 ein Referenzsignal für den Lock-InVerstärker 36.

Der Referenzdetektor 34′ erzeugt ein Referenzphotosignal 35′, das einen Referenz-Lock-In-Verstärker 36′ beaufschlagt, der ausgangsmäßig mit einem weiteren Eingang der Steuer- und Auswerteschaltung 37 verbunden ist. Die Steuer- und Auswerteschaltung 37 gibt über die zweite Referenzsteuerleitung 39′ ein Referenzsignal für den Referenz-Lock-In-Verstärker 36′.

Das in der Steuer- und Auswerteschaltung 37 erzeugte Regelsignal 40 wird analog den ersten Ausführungsbeispielen verwendet.

Bei Abwesenheit optisch drehender Stoffe im Referenzkanal, d.h. in der Referenzflüssigkeit 11′, kann die Intensität der als Strahlungsquelle dienenden Lumineszenzdiode 1 über eine automatisch regelbare Versorgungsschaltung 65 derart abgeglichen werden, daß das Referenzphotosignal 35′ des Referenzdetektors 34′ für beide jeweils einzeln leuchtende Lumineszenzdioden 1 und 21 gleich ist. Ein solcher automatischer Abgleich ist bei langzeitigem Einsatz des Polarimeters von besonderem Vorteil, da dann mit Alterungseffekten der Strahlungsquellen gerechnet werden muß.

Die Lumineszenzdioden 1 und 21 strahlen intermittierend mit Frequenzen im Bereich von unter zehn Hertz bis zu einigen Kilohertz. Das dann entstehende Lock-In-Signal gestattet in der Versorgungsschaltung 65 im Referenzkanal den Signalabgleich durch Nullung.

Die Emission des Lichts ist vorzugsweise noch mit einer zweiten Modulation überlagert, die mit einer Frequenz aus einem Bereich von z.B. ein Kilohertz bis hundert Kilohertz durchgeführt wird. Die zweite Frequenz sollte mindestens eine dezimale Größenordnung größer sein als die oben genannte erste Modulationsfrequenz. Die Modulation liegt z.B. im schnellen Ein/Ausschalten der jeweilig betriebenen Lumineszenzdiode 1 oder 21. Dann erzeugen die Lumineszenzdioden 1 und 21 über den Referenz-Lock-In-Verstärker 36′ für jede Lichtquelle ein eigenes Signal, wovon eines dieser Signale mit Hilfe der automatisch regelbaren Versorgungsschaltung 65 auf das andere abgestimmt wird.

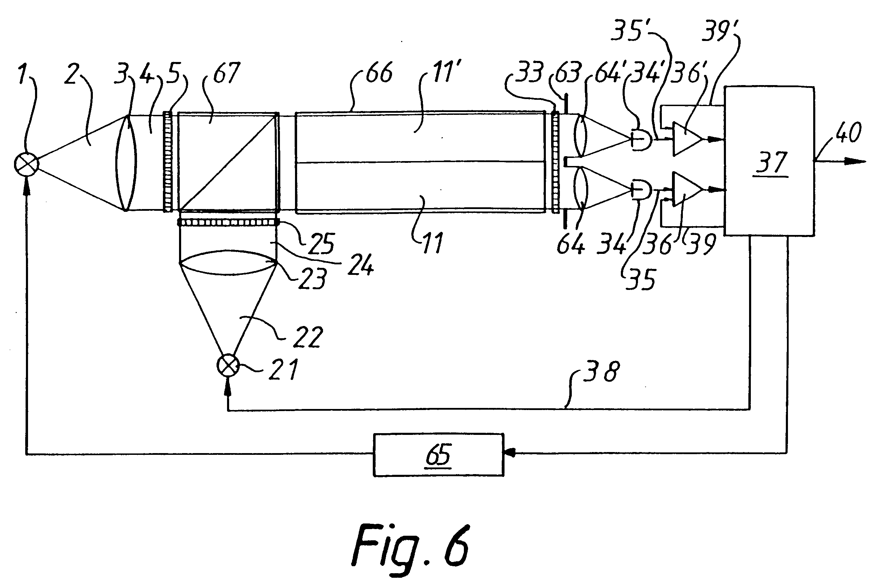

Die Figur 6 zeigt schematisch eine Seitenansicht einer Vorrichtung zur quantitativen Bestimmung optisch aktiver Substanzen in einer menschlichen Körperflüssigkeit gemäß einem vierten Ausführungsbeispiel der Erfindung. Der von der ersten Lumineszenzdiode ausgehende erste divergente Lichtstrahl 2 wird von der ersten Kollimatorlinse 3 zu dem ersten parallelen Lichtstrahl 4 gebündelt, der durch das erste Polarisationsfilter 5 und einen Polarisationsstrahlteilerwürfel 67 als erster abbildender Lichtstrahl 60 in die Doppelkammerküvette 66 eintritt. Der von der zweiten Lumineszenzdiode ausgehende zweite divergente Lichtstrahl 22 wird von der zweiten Kollimatorlinse 23 zu dem zweiten parallelen Lichtstrahl 24 gebündelt, der durch das zweite Polarisationsfilter 25 hindurchtritt und mit dem Polarisationsstrahlteilerwürfel 67 zum zweiten abbildenden Lichtstrahl 61 in die Doppelkammerküvette 66 umgelenkt wird.

Beide Lichtstrahlen 60 und 61 fluchten miteinander und weisen vorzugsweise die gleiche Strahltaille auf. Für den Fall geringerer Anforderungen an den Polarisationsgrad der Lichtstrahlen 60 und 61 kann der Polarisationsstrahlteilerwürfel 67 alleine benutzt werden und auf die Polarisationsfilter 5 und 25 verzichtet werden. Es ist auch möglich, einen einfachen üblichen nicht depolarisierenden Strahlteiler einzusetzen, wenn die Polarisationsfilter 5 und 25 verwendet werden. Der Winkel zwischen den Polarisationsrichtungen der beiden Lichtstrahlen 60 und 61 ist wie in den vorherigen Ausführungsbeispielen, insbesondere zu je 45°, gewählt.

Die Doppelkammerküvette 66 enthält zwei Kammern, die mit dem Dialysat 11 und der Referenzflüssigkeit 11′ gefüllt sind. Der weitere Strahlengang und die Auswertung der erhaltenen Photosignale 35 und 35′ erfolgt analog wie im dritten Ausführungsbeispiel. Das vierte Ausführungsbeispiel weist den Vorteil auf, daß die Meß- und Referenzstrahlengänge identisch sind, wodurch beispielsweise intensitätsschwächende Ablagerungen im Meßstrahlengang die Meßergebnisse nicht verfälschen.

## Patentansprüche

1. Verfahren zur quantitativen Bestimmung der Konzentration optisch aktiver Substanzen, insbesondere von Glukose in der Körperflüssigkeit eines Patienten, durch Polarimetrie unter Erzeugung eines Vergleichssignals, insbesondere eines Differenz- oder Quotientensignals, mit einer ersten Lichtquelle (1), mit der ein erstes linear polarisiertes Lichtbündel erzeugt wird, das die zu analysierende Substanz durchstrahlt und über einen Analysator (33) einen Detektor (34) beaufschlagt, **dadurch gekennzeichnet**, daß ein zweites linear polarisiertes Lichtbündel mit Hilfe einer zweiten Lichtquelle (21) erzeugt wird, das ebenfalls die zu analysierende Substanz durchstrahlt und über den Analysator (33) den Detektor (34) beaufschlagt, daß als feste Polarisationsrichtung des zweiten linear polarisierten Lichtbündels eine Polarisationsrichtung gewählt wird, die um einen vorbestimmten Winkel von der festen Polarisationsrichtung des ersten linear polarisierten Lichtbündels abweicht, daß die erste und zweite Lichtquelle im Wechsel mit einer Umschaltfrequenz ein- und ausgeschaltet werden und daß die von dem Detektor (34) erzeugten zu den ersten und zweiten linear polarisierten Lichtbündeln gehörigen Meßsignale zur Erzeugung eines oder mehrerer Vergleichssignale, insbesondere von Differenz- oder Quotientensignalen, herangezogen werden.

2. Verfahren nach Anspruch 1, dadurch gekennzeichnet, daß die Polarisationsrichtung des Analysators (33) im wesentlichen rechtwinklig zu den Polarisationsrichtungen des ersten und zweiten Lichtbündels ausgerichtet wird.

3. Verfahren nach Anspruch 1, dadurch gekennzeichnet, daß der vorbestimmte Winkel zwischen den Polarisationsrichtungen des ersten und zweiten Lichtbündels 90° beträgt und daß die Polarisationsrichtung des Analysators (33) im wesentlichen um 45° zu der Polarisationsrichtung des ersten oder des zweiten Lichtbündels verdreht wird.

4. Verfahren nach einem der Ansprüche 1 bis 3, dadurch gekennzeichnet, daß die Lichtquellen mit einer Modulationsfrequenz moduliert werden, um das Signal des Detektors (34) phasenempfindlich nachzuweisen, wobei die Umschaltfrequenz um mindestens eine dezimale Größenordnung kleiner als die Modulationsfrequenz ist.

5. Vorrichtung zur Durchführung des Verfahrens nach einem der Ansprüche 1 bis 4, dadurch gekennzeichnet, daß mindestens zwei Lichtquellen (1,21) vorgesehen sind, die im Wechsel der Umschaltfrequenz ein- und ausgeschaltet sind, daß die Lichtbündel der Lichtquellen (1,21) in verschiedenen Polarisationsrichtungen linear polarisiert sind und die Substanz (11) durchstrahlen, deren Konzentration an optisch drehenden Stoffen gemessen wird, wobei die Lichtbündel nach dem Durchtritt durch einen Analysator (33) auf einen Detektor (34) abgebildet werden, dessen Photosignalausgang an eine Steuer- und Auswerteelektronik (36,37) angeschlossen ist, in der die Meßsignale zwischenspeicherbar sind und die eine Rechenschaltung zur Bildung eines Vergleichssignals, insbesondere eines Quotienten- oder Differenzsignals aufweist, das ein der Konzentration der Substanz zugeordnetes Regelsignal (40) darstellt.

6. Vorrichtung nach Anspruch 5, dadurch gekennzeichnet, daß als Lichtquellen (1,21) Lumineszenzdioden vorgesehen sind, in deren Lichtweg Polarisationsfilter (5, 25) angeordnet sind.

7. Vorrichtung nach Anspruch 5, dadurch gekennzeichnet, daß als Lichtquellen (1,21) Halbleiterlaserdioden vorgesehen sind.

8. Vorrichtung nach Anspruch 7, dadurch gekennzeichnet, daß in deren Lichtweg Polarisationsfilter (5, 25) angeordnet sind.

9. Vorrichtung nach einem der Ansprüche 5 bis 8, dadurch gekennzeichnet, daß eine Küvette (10) zur Aufnahme der flüssigen Substanz (11) vorgesehen ist, deren Konzentration an optisch drehenden Stoffen erfaßt werden soll, und daß der Strahlengang der Lichtquellen in Längsrichtung der Küvette (10) ausgerichtet ist, wobei die Vorderwand (12) sowie die Rückwand (13) der Küvette (10) transparent sind.

10. Vorrichtung nach Anspruch 9, dadurch gekennzeichnet, daß die Lichtquellen (1,21) mit den zugehörigen Kollimatorlinsen (3,23) in Verlängerung der Längsachse der Küvette (10) derart angeordnet sind, daß die polarisierten Lichtbündel (9,29,60,61) nach dem Durchgang durch die die Vorderwand (12) der Küvette (10) bildenden Polarisationsfilter (5,25) und durch den die Rückwand (13) der Küvette (10) bildenden Analysator (33) auf den Detektor (34) abgebildet werden.

11. Vorrichtung nach Anspruch 9, dadurch gekennzeichnet, daß in Verlängerung der Längsachse der Küvette (10) ein Strahlteilerwürfel (67) angeordnet ist, daß die erste Lichtquelle (1) mit der ersten Kollimatorlinse (3) in Verlängerung der Längsachse der Küvette (10) angeordnet ist, daß die zweite Lichtquelle (21) mit der zweiten Kollimatorlinse (23) im wesentlichen rechtwinklig zum Strahlteilerwürfel (67) angeordnet ist, daß die polarisierten Lichtbündel (60,61) nach dem Durchgang durch den Strahlteilerwürfel (67) die Vorderwand (12) der Küvette (10) beaufschlagen und jeweils zum Teil durch die mit der zu analysierenden Substanz (11) füllbaren Kammer bzw. durch die mit der Referenzflüssigkeit (11′) füllbaren Kammer hindurchtreten und durch den die Rückwand (13) der Küvette (10) bildenden Analysator (33) auf den Detektor (34) bzw. auf den Referenzdetektor (34′) abgebildet werden.

12. Vorrichtung nach Anspruch 11, dadurch gekennzeichnet, daß zwischen den Kollimatorlinsen (3 bzw. 23) und dem Strahlteilerwürfel (67) jeweils ein Polarisationsfilter (5 bzw. 25) angeordnet ist.

13. Vorrichtung nach Anspruch 11 oder 12, dadurch gekennzeichnet, daß der Strahlteilerwürfel (67) ein Polarisationsstrahlteilerwürfel ist.

14. Vorrichtung nach einem der Ansprüche 9 bis 13, dadurch gekennzeichnet, daß die Küvette (10) eine zweite Kammer umfaßt, die parallel zu der Kammer mit der flüssigen Substanz (11) verläuft, deren Konzentration an optisch drehenden Stoffen erfaßt werden soll, und wobei die zweite Kammer sich in Längsrichtung der Küvette (10) von deren Vorderwand (12) bis zu deren Rückwand (13) erstreckt und sie zur Aufnahme einer Referenzflüssigkeit (11′) vorgesehen ist, daß die Lichtbündel der Lichtquellen (1,21) die Referenzflüssigkeit (11′) durchstrahlen, wobei die Lichtbündel nach dem Durchtritt durch den Analysator (33) auf einen Referenzdetektor (34′) abgebildet werden, dessen Photosignalausgang an die Steuer- und Auswerteelektronik (36′,37) angeschlossen ist, in der die Meßsignale zwischenspeicherbar sind und die eine Rechenschaltung zur Bildung eines Vergleichssignals aufweist, das ein der Lichtausbeute der Lichtquellen (1,21) zugeordnetes Regelsignal darstellt, das in eine Versorgungsschaltung (65) zur Versorgung einer der Lichtquellen (1,21) einspeisbar ist.

## Claims

1. Method for the quantitative determination of the concentration of optically active substances, preferably of glucose in the body fluid of a patient, by means of polarimetry by the generation of a comparison signal, preferably a difference or quotient signal, having a first light source (1) by means of which a first linearly polarized light bundle is generated, which is transmitted through the substance to be analysed and falls on a detector (34) via an analyser (33), characterized in that a second linearly polarized light bundle is generated with the aid of a second light source (21), and is likewise transmitted through the substance to be analysed and falls on the detector (34) via the analyser (33), in that, as the fixed direction of polarization of the second linearly polarized light bundle, a direction of polarization is chosen which deviates from the fixed direction of polarization of the first linearly polarized light bundle by a predetermined angle, in that the first and second light sources are alternately switched on and off at a switching frequency, and in that the measurement signals generated by the detector (34) and belonging to the first and second linearly polarized light bundles are used to generate one or more comparison signals, preferably difference or quotient signals.

2. Method according to Claim 1, characterized in that the direction of polarization of the analyser (33) is aligned essentially at right angles to the direction of polarization of the first and second light bundles.

3. Method according to Claim 1, characterized in that the predetermined angle between the direction of polarization of the first and second light bundles amounts to 90° and that the direction of polarization of the analyser (33) is rotated essentially by 45° relative to the direction of polarization of the first or the second light bundle.

4. Method according to one of Claims 1 to 3, characterized in that the light source is modulated at a modulation frequency in order to detect the signal from the detector (34) in a phase-sensitive manner, the switching frequency being at least a decimal order of magnitude less than the modulation frequency.

5. Device for carrying out the method according to one of Claims 1 to 4, characterized in that at least two light sources (1,21) are provided, which are alternately switched on and off at the switching frequency, in that the light bundles from the light sources (1,21) are linearly polarized in different directions of polarization and are transmitted through the substance (11) of which the concentration of optically rotating materials is being measured, the light bundles, after passing through an analyser (33), being projected onto a detector (34) of which the photoelectric signal output is connected to control and evaluation electronics (36,37), in which the measurement signals may be stored temporarily and which have a processor circuit for the formation of a comparison signal, preferably a quotient or difference signal, which represents a control signal (40) associated with the concentration of the substance.

6. Device according to Claim 5, characterized in that, as the light sources (1,21), light-emitting diodes are provided, in whose light paths polarization filters (5, 25) are arranged.

7. Device according to Claim 5, characterized in that, as light sources (1,21), semiconductor laser diodes are provided.

8. Device according to Claim 7, characterized in that polarization filters (5, 25) are arranged in their light paths.

9. Device according to one of Claims 5 to 8, characterized in that a cuvette (10) is provided to receive the liquid substance (11) of which the concentration of optically rotating substances is to be measured, and that the beam path of the light sources is aligned with the longitudinal direction of the cuvette (10), the front wall (12) and the rear wall (13) of the cuvette (10) being transparent.

10. Device according to Claim 9, characterized in that the light sources (1,21) are arranged, with the collimator lenses (3,23) associated, on an extension of the longitudinal axis of the cuvette (10) that after passage through the polarization filters (5, 25) forming the front wall (12) of the cuvette (10) and through the analyser (33) forming the rear wall (13) of the cuvette (10) rear wall (13) of the cuvette (10), the polarized light bundles (9,29,60,61) are projected onto the detector (34).

11. Device according to Claim 9, characterized in that a beam-splitter cube (67) is arranged on an extension of the longitudinal axis of the cuvette (10), in that the first light source (1) with the first collimator lens (3) is arranged on an extension of the longitudinal axis of the cuvette (10), in that the second light source (21) with the second collimator lens (23) is arranged essentially at right angles to the beam-splitter cube (67), in that the polarized light bundles (60,61), after passing through the beam-splitter cube (67), fall on the front wall (12) of the cuvette (10) and pass respectively partly through the chamber which can be filled with the substance (11) to be analysed and partly through the chamber which can be filled with the reference liquid (11′) and are projected respectively onto the detector (34) (10) and onto the reference detector (34′) by the analyser (33) forming the rear wall (13) of the cuvette.

12. Device according to Claim 11, characterized in that polarization filters (5 and 25) are arranged between the respective collimator lenses (3 and 23) and the beam-splitter cube (67).

13. Device according to Claim 11 or 12, characterized in that the beam-splitter cube (67) is a polarization beam-splitter cube.

14. Device according to one of Claims 9 to 13, characterized in that the cuvette (10) includes a second chamber which runs parallel to the chamber with the liquid substance (11) of which the concentration of optically rotating materials is to be registered, the second chamber extending in the longitudinal direction of the cuvette (10) from its front wall (12) up to its rear wall (13) and being provided to receive a reference liquid (11′), in that the light bundles from the light sources (1,21) are transmitted through the reference liquid, the light bundles after passing through the analyser (33) being projected onto a reference detector (34′) of which the photoelectric signal output is connected to the control and evaluation electronics (36′,37), in which the measurement signals may be temporarily stored and which have a processor circuit for the formation of a comparison signal which represents a control signal which is associated with the light yield of the light sources (1,21) and may be fed to a supply circuit (65) for supplying one of the light sources (1,21).

## Revendications

1. Procédé de détermination quantitative de la concentration de substances actives du point de vue optique, notamment du glucose dans le liquide corporel d'un patient, par polarimétrie moyennant la production d'un signal de référence, notamment un signal de différence ou de quotient, comportant une première source de lumière (1) au moyen de laquelle est produit un premier faisceau de lumière polarisé linéairement qui traverse la substance à analyser et charge, par l'intermédiaire d'un analyseur (33), un détecteur (34), caractérisé par le fait qu'un second faisceau de lumière polarisé linéairement est produit à l'aide d'une seconde source de lumière (21), qui traverse également la substance à analyser et charge le détecteur (34) par l'intermédiaire de l'analyseur (33), qu'on choisit, comme direction fixe de polarisation du second faisceau de lumière polarisé linéairement, une direction de polarisation qui s'écarte, d'un angle prédéterminé de la direction fixe de polarisation du premier faisceau de lumière polarisé linéairement, que les première et seconde sources de lumière sont branchées et éteintes alternativement selon une fréquence de commutation et que les signaux de mesure produits par le détecteur (34) et associés aux premier et second faisceaux de lumière polarisés linéairement, sont utilisés pour produire un ou plusieurs signaux de référence, notamment des signaux de différence ou de quotient.

2. Procédé suivant la revendication 1, caractérisé par le fait que la direction de polarisation de l'analyseur (33) est dirigée sensiblement perpendiculairement aux directions de polarisation des premier et second faisceaux de lumière.

3. Procédé suivant la revendication 1, caractérisé par le fait que l'angle prédéterminé entre les directions de polarisation des premier et second faisceaux de lumière est égal à 90° et que la direction de polarisation de l'analyseur (33) est pivotée sensiblement de 45° par rapport à la direction de polarisation du premier ou du second faisceau de lumière.

4. Procédé suivant l'une des revendications 1 à 3, caractérisé par le fait que les sources de lumière sont modulées avec une fréquence de modulation, pour la détection, d'une manière sensible à la phase du signal du détecteur (34), la fréquence de commutation étant inférieure, au moins d'un ordre de grandeur décimal, à la fréquence de modulation.

5. Dispositif pour la mise en oeuvre du procédé suivant l'une des revendications 1 à 4, caractérisé par le fait qu'il est prévu au moins deux sources de lumière (1,21), qui sont allumées et éteintes au rythme de la fréquence de commutation, que les faisceaux de lumière des sources de lumière (1,21) sont polarisés linéairement dans différentes directions de polarisation et traversent la substance (11), dans laquelle est mesurée la concentration de matières qui produisent une rotation optique, l'image des faisceaux de lumière étant formée, après passage par un analyseur (33), sur un détecteur (34), dont le signal de sortie optique est envoyé à une unité électronique de commande et d'évaluation (36,37), dans laquelle les signaux de mesure peuvent être mémorisés temporairement et qui possède un circuit de calcul servant à former un signal de référence, notamment un signal de quotient ou de différence, qui représente un signal de régulation (40) associé à la concentration de la substance.

6. Dispositif suivant la revendication 5, caractérisé par le fait qu'il est prévu, comme sources de lumière (1, 21), des diodes à luminescence, dans la trajectoire de lumière desquelles sont disposés des filtres de polarisation (5,25).

7. Dispositif suivant la revendication 5, caractérisé par le fait qu'il est prévu des diodes laser à semiconducteurs en tant que sources de lumière (1,21).

8. Dispositif suivant la revendication 7, caractérisé par le fait que les filtres de polarisation (5,25) sont disposés dans leurs trajectoires de lumière.

9. Dispositif suivant l'une des revendications 5 à 8, caractérisé par le fait qu'il est prévu une cuvette (10) servant à recevoir la substance liquide (11) dans laquelle on doit détecter la concentration de substances produisant une rotation optique, et que la trajectoire du rayonnement des sources de lumière est orienté dans la direction longitudinale de la cuvette (10), la paroi avant (12) ainsi que la paroi arrière (13) de la cuvette (10) étant transparentes.

10. Dispositif suivant la revendication 9, caractérisé par le fait que les sources de lumière (1,21) pourvues des lentilles de collimation associées (3,23) sont disposées dans le prolongement de l'axe longitudinal de la cuvette (10), et que l'image des faisceaux de lumière polarisés (9,29,60,61) est formée sur le détecteur (34), après leur passage par les filtres de polarisation (5,25) formant la paroi avant (12) de la cuvette (10), et l'analyseur (33), qui forme la paroi arrière (13) de la cuvette (10).

11. Dispositif suivant la revendication 9, caractérisé par le fait qu'un cube diviseur de faisceau (67) est disposé dans le prolongement de l'axe longitudinal de la cuvette (10), que la première source de lumière (1), pourvue de la première lentille de collimation (30), est disposée dans le prolongement de l'axe longitudinal de la cuvette (10), que la seconde source de lumière (21) est disposée, ainsi que la seconde lentille de collimation (23), sensiblement perpendiculairement au cube diviseur de faisceau (67), que les faisceaux de lumière polarisés (60,61) rencontrent la paroi avant (12) de la cuvette (10) après avoir traversé le cube diviseur de faisceau (67) alors que chacun d'eux traverse respectivement partiellement la chambre qui peut être remplie avec la substance (11) à analyser et la chambre qui peut être remplie avec le liquide de référence (11′), l'image de ces faisceaux est formée par l'analyseur (33), qui constitue la paroi arrière (13) de la cuvette (10), sur le détecteur (34) ou sur le détecteur de référence (34′).

12. Dispositif suivant la revendication 11, caractérisé par le fait que respectivement un filtre de polarisation (5 ou 25) est disposé entre les lentilles de collimation (3 ou 23) et le cube diviseur de faisceau (67).

13. Dispositif suivant la revendication 11 ou 12, caractérisé par le fait que le cube diviseur de faisceau (67) est un cube diviseur de faisceau à polarisation.

14. Dispositif suivant l'une des revendications 9 à 13, caractérisé par le fait que la cuvette (10) comprend une seconde chambre, qui est parallèle à la chambre contenant la substance liquide (11), dans laquelle il faut déterminer la concentration de substances produisant une rotation optique, et que la seconde chambre s'étend dans la direction longitudinale de la cuvette (10) depuis son bord avant (12) jusqu'à sa paroi arrière (13) et destinée à recevoir un liquide de référence (11′), que les faisceaux de lumière des sources de lumière (1,21) traversent le liquide de référence (11′), l'image des faisceaux de lumière étant formée, après qu'ils passagé par l'analyseur (33), sur un détecteur de référence (34′) dont la sortie du signal optique est raccordée à l'unité électronique de commande et d'évaluation (36′,37), dans laquelle les signaux de mesure peuvent être mémorisés temporairement et qui possède un circuit de calcul servant à former un signal de référence qui représente un signal de régulation associé au rendement lumineux des sources de lumière (1,21) et qui peut être injecté dans un circuit d'alimentation (65) servant à alimenter l'une des sources de lumière (1,21).
